# EUROPEAN PATENT APPLICATION

(11) **EP 4 400 582 A1**
(43) Date of publication of application: **17.07.2024**
(21) Application number: 21956476.2
(22) Date of filing: 16.09.2021
(51) Int. Cl.: C12N 5/09, C12N 5/071

(54) **CULTURE MEDIUM FOR HEPATOMA ORGANOID CULTURE, HEPATOMA ORGANOID CULTURE METHOD, AND APPLICATION OF HEPATOMA ORGANOID**

(30) Priority: 08.09.2021 CN 202111048845
(71) Applicant: Precedo Pharmaceuticals Co., Ltd., Hefei, Anhui 230094 (CN)
(72) Inventor: LIU, Qing Song, Hefei, Anhui 230031 (CN); WANG, Wen Liang, Hefei, Anhui 230031 (CN); HUANG, Tao, Hefei, Anhui 230031 (CN); CHEN, Cheng, Hefei, Anhui 230031 (CN)
(74) Representative: Abel & Imray LLP
(86) International application number: PCT/CN2021/118657
(87) International publication number: WO 2023/035299

(57) **Abstract**

A culture medium for hepatoma organoid culture, comprising an MST1/2 kinase inhibitor, at least one cell culture additive selected from N2 and B27, a hepatocyte growth factor, an ITS cell culture additive, Y27632, dexamethasone, Neuregulin-1, insulin, an epidermal cell growth factor, GlutaMAX, and non-essential amino acids. The application further relates to a hepatoma organoid culture method and an application thereof. By using the culture medium for hepatoma organoid, effective and rapid expansion of the hepatoma organoid can be achieved, and the organoid obtained by such expansion maintains the pathological characteristics of a patient, improves the culture success rate and the expansion rate of the hepatoma organoid, and provides a research basis for individualized treatment of the patient.

## Description

### Technical Field

The invention relates to the field of biotechnology, and in particular to a culture medium for hepatoma organoid culture, a method for culturing hepatoma organoid using the culture medium, and the application thereof in the efficacy evaluating and screening of drugs.

### Background of the Invention

In recent years, postoperative adjuvant chemotherapy for liver cancer, including postoperative TACE treatment, oral medication treatment, etc., has gradually gained the attention and recognition of clinical doctors as a new type of adjuvant treatment. However, due to the lack of standardized chemotherapy regimens, conventional chemotherapies based on experiences may ignore individual differences and lead to blindness, and thus, the efficacy has always been unsatisfied, with the efficacy rate of less than 20% in monotherapy or combination therapy (Jindal A, Thadi A, Shailubhai K. Hepatocellular Carcinoma: Etiology and Current and Future Drugs [J]. J Clin Exp Hepatol, 2019, 9(2): 221-232). Although emerging targeted drugs have reduced toxic side-effects to a certain extent, the quantity of drugs is too small, the treatment cost is expensive, and the effectiveness varies among individuals, making it difficult to meet the treatment needs of most patients. Due to the lack of an effective liver cancer drug sensitivity testing system, precise chemotherapy cannot be achieved. Therefore, matching the *in vitro* drug sensitivity results of liver cancer with clinical *in vivo* reactions becomes the key to treatment.

Traditional clinical drug sensitivity testing mostly uses two-dimensional cell culture. However, two-dimensional cultured cells can only simulate the physiological conditions of tissue to a limited extent, without real tissue structure *in vivo,* which may prone to low differentiation levels and loss of cell physiological functions, making it difficult to predict actual clinical results from the obtained experimental results. Organoids belong to three-dimensional (3D) cell cultures, which are mainly derived from human embryonic stem cells, induced pluripotent stem cells, and adult stem cells with differentiation ability. Endogenous tissue stem cells, which play an important role in maintaining the functional morphology of each organ, exist in different tissues and organs. These stem cells can self organize and form a mini structure with a diameter of only a few millimeters under certain induction conditions *in vitro.* Tumor organoids are miniature 3D tumor cell models cultured in the laboratory from primary tumors taken from patient's body. Tumor organoids can highly simulate the characteristics of the source tumor tissue, preserving tumor heterogeneity among individuals, and can be used for functional testing, such as high-throughput drug screening and personalized precision therapy.

Currently, culture methods for hepatoma organoid often use basic culture media (DMEM or DMEM/F12), R-spindin-1, Noggin, and some expensive protein factors, resulting in higher costs for organoid culture; and the complex operation and the difficulty of this technology have limited its large-scale commercial applications. Therefore, it is necessary to develop a low-cost, simple, and of high success rate culture method and culture medium for organoid.

### Summary of the Invention

In order to solve the aforementioned technical problems, the invention provides a culture medium and culture method for rapidly expanding hepatoma organoid *in vitro.*

One aspect of the invention is to provide a culture medium for hepatoma organoid, comprising an MST1/2 kinase inhibitor, at least one cell culture additive selected from N2 and B27, a hepatocyte growth factor, an ITS cell culture additive, Y27632, dexamethasone, Neuregulin-1, insulin, an epidermal cell growth factor, GlutaMAX, and non-essential amino acids. Wherein the MST1/2 kinase inhibitor comprises a compound of Formula (I) or a pharmaceutically acceptable salt, or a solvate thereof, 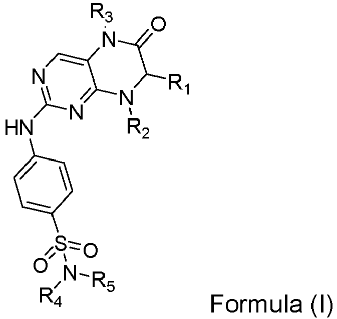 Wherein,
R₁ is selected from C1-C6 alkyl, C3-C6 cycloalkyl, C4-C8 cycloalkylalkyl, C2-C6 spirocycloalkyl, and aryl (e.g., phenyl and naphthyl, etc.) optionally substituted with 1-2 independent R₆, aryl C1-C6 alkyl (e.g., phenylmethyl, etc.) optionally substituted with 1-2 independent R₆ and heteroaryl (e.g., thienyl, etc.) optionally substituted with 1-2 independent R₆;
R₂ and Rs are each independently selected from C1-C6 alkyl, preferably C1-C3 alkyl, more preferably methyl;
R₄ and R₅ are each independently selected from hydrogen, C1-C6 alkyl, C3-C6 cycloalkyl, C4-C8 cycloalkylalkyl, hydroxyl C1-C6 alkyl, C1-C6 haloalkyl, C1-C6 alkylamino C1-C6 alkyl, C1-C6 alkoxy C1-C6 alkyl, and C3-C6 heterocyclyl C1-C6 alkyl (the heterocyclyl is selected from e.g., piperidyl, tetrahydropyranyl, etc.);
R₆ is selected from halogen (preferably fluoro and chloro, more preferably fluoro), C1-C6 alkyl (preferably methyl), C1-C6 alkoxy (preferably methoxy), and C1-C6 haloalkyl (preferably trifluoromethyl).

In a preferable embodiment, the MST1/2 kinase inhibitor comprises a compound of Formula (Ia) or a pharmaceutically acceptable salt, or a solvate thereof, 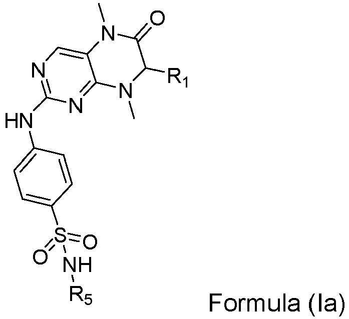 Wherein,
R₁ is selected from C1-C6 alkyl, phenyl optionally substituted with 1-2 independent R₆, thienyl optionally substituted with 1-2 independent R₆, and phenylmethyl optionally substituted with 1-2 independent R₆; more preferably, R₁ is phenyl optionally substituted with 1-2 independent R₆;
R₅ is selected from hydrogen, C1-C6 alkyl, and C3-C6 cycloalkyl; more preferably, R₅ is hydrogen;
R₆ is independently selected from halogen, C1-C6 alkyl, and C1-C6 haloalkyl; more preferably, R₆ is fluoro, methyl or trifluoromethyl.

Preferably, the MST1/2 kinase inhibitor is at least one selected from the following compounds or a pharmaceutically acceptable salt, or a solvate thereof.

| | | | |
|---|---|---|---|
| 1 | 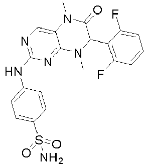 | 2 | 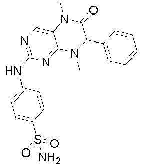 |
| 3 | 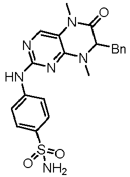 | 4 | 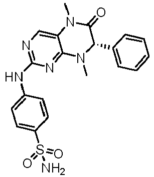 |
| 5 | 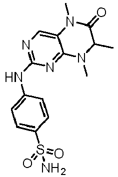 | 6 | 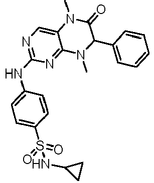 |
| 7 | 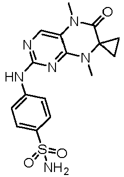 | 8 | 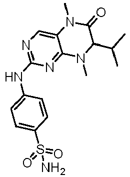 |
| 9 | 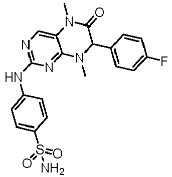 | 10 | 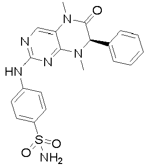 |
| 11 | 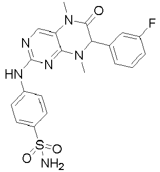 | 12 | 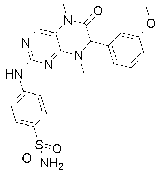 |
| 13 | 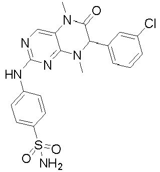 | 14 | 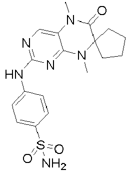 |
| 15 | 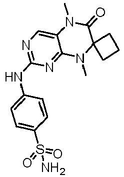 | 16 | 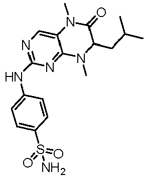 |
| 17 | 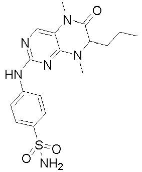 | 18 | 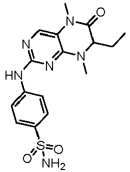 |
| 19 | 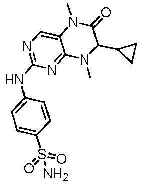 | 20 | 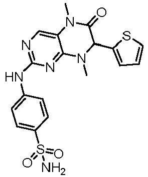 |
| 21 | 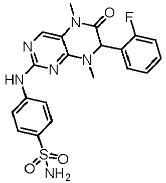 | 22 | 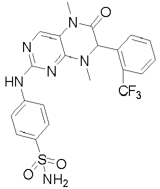 |
| 23 | 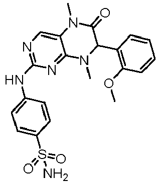 | 24 | 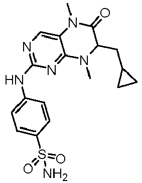 |
| 25 | 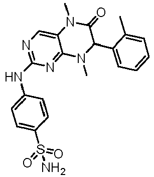 | 26 | 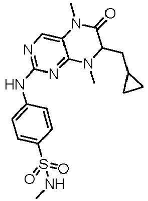 |
| 27 | 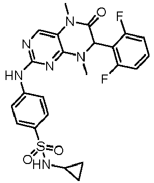 | 28 | 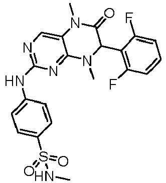 |
| 29 | 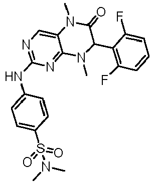 | 30 | 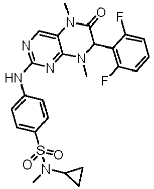 |
| 31 | 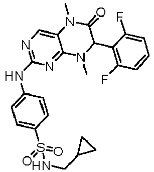 | 32 | 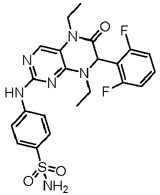 |
| 33 | 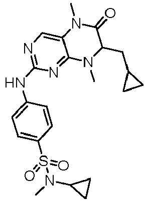 | 34 | 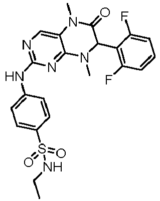 |
| 35 | 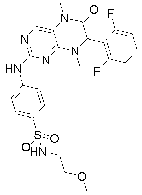 | 36 | 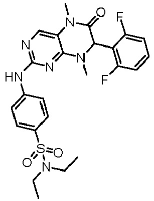 |
| 37 | 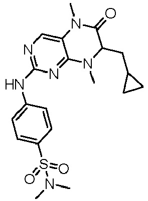 | 38 | 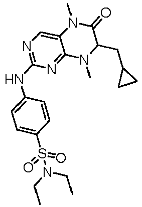 |
| 39 | 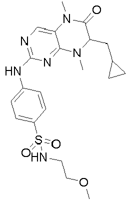 | 40 | 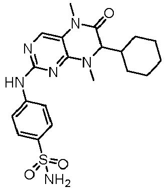 |
| 41 | 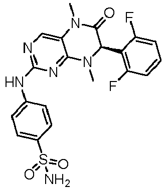 | 42 | 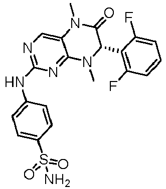 |
| 43 | 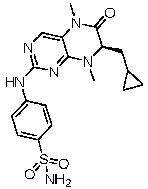 | 44 | 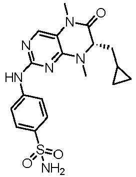 |
| 45 | 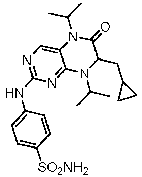 | 46 | 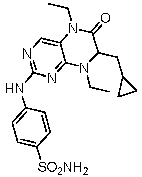 |
| 47 | 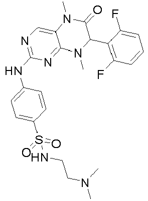 | 48 | 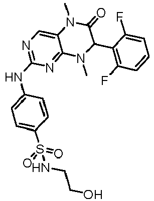 |
| 49 | 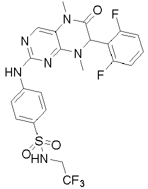 | 50 | 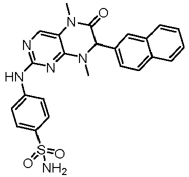 |
| 51 | 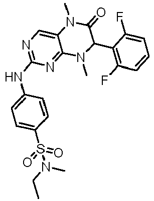 | 52 | 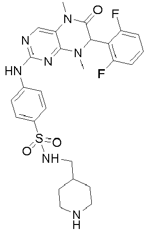 |
| 53 | 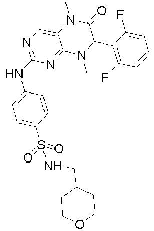 | 54 | 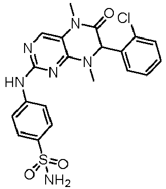 |
| 55 | 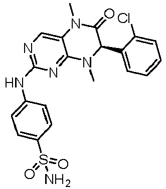 | 56 | 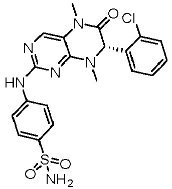 |
| 57 | 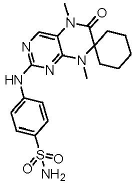 | 58 | 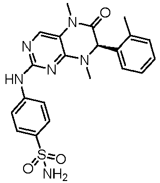 |
| 59 | 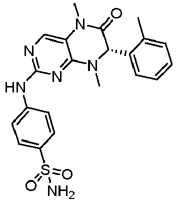 | | |

Most preferably, the MST1/2 kinase inhibitor of the invention is Compound 1.

In the embodiment of the invention, the amounts of the components in the culture medium of the invention satisfies any one or more or all of the following conditions:
(1) the concentration of the MST1/2 kinase inhibitor is 2.5-10 µM);
(2) the volume ratio of the B27 or N2 cell culture additive in the culture medium is 1:25-1:100;
(3) the concentration of the hepatocyte growth factor is 1-25 ng/mL;
(4) the volume ratio of the ITS cell culture additive in the culture medium is 1:30-1:300;
(5) the concentration of Y27632 is 3-30 µM);
(6) the concentration of dexamethasone is 0.1-1 µM);
(7) the concentration of Neuregulin-1 is 1-25 ng/mL;
(8) the concentration of insulin is 1-10 µg/mL;
(9) the concentration of the epidermal cell growth factor is 2-18 ng/mL;
(10) the volume ratio of GlutaMAX in the culture medium is 1:30-1:300;
(11) the non-essential amino acids are one or more selected from the group consisting of glycine, alanine, asparagine, aspartic acid, glutamic acid, proline and serine, and the concentration of the non-essential amino acids is 50-200 µM.

In the embodiment of the invention, the culture medium further comprises an initial medium selected from the group consisting of DMEM/F12, DMEM, F12 or RPMI-1640; and one or more antibiotics selected from the group consisting of streptomycin/penicillin, amphotericin B and Primocin.

In a preferred embodiment, in case of using streptomycin/penicillin as the antibiotics, the concentration range of streptomycin is 25-400 µg/mL, the concentration range of penicillin is 25-400 U/mL; in case of using amphotericin B as the antibiotics, the concentration range is 0.25-4 µg/mL; and in case of using Primocin as the antibiotics, the concentration range is 25-400 µg/mL.

The invention also provides a method for culturing hepatoma organoid. In the method for culturing hepatoma organoid of the invention, the hepatoma organoid is cultured using the culture medium for hepatoma organoid of the invention.

The method for culturing hepatoma organoid of the invention comprises the following steps.
1. Isolating samples from solid liver cancer tissue to obtain primary liver cancer cells. The process includes the following steps:
   (1) liver cancer tissue samples are separated and added with a 1:3 ratio of basic medium and tissue digestion solution (the amount of tissue digestion solution added is about 10 mL tissue digestion solution for 1 g of tumor tissue), and placed in a constant-temperature shaker for digestion, at a digestion temperature of 4-37°C, a rotation rate of 200-350 rpm, for a digestion time of 3-6 hours;
   (2) after digestion, the supernatant is discarded after centrifugation with a centrifugation speed of 1200-1600 rpm for a centrifugation time of 2-6 minutes.

Wherein, the formulation of the basic medium comprises an initial medium selected from the group consisting of DMEM/F12, DMEM, F12 or RPMI-1640; and one or more antibiotics selected from the group consisting of streptomycin/penicillin, amphotericin B and Primocin. The formulation of the tissue digestion solution comprises 1640 medium, collagenase II (1-2 mg/mL), collagenase IV (1-2 mg/mL), DNase (50-100 U/mL), hyaluronidase (0.5-1 mg/mL), calcium chloride (1-5 mM), bovine serum albumin BSA (5-10 mg/mL).

2. Preparing the culture medium for hepatoma organoid of the invention, and culturing the primary liver cancer cells obtained in the above step.

The primary liver cancer cells obtained in above step 1 are resuspended with the culture medium for hepatoma organoid of the invention and counted. The cell density is diluted to 5-10×10⁶ cells/mL, and the diluted cell suspension is added with an equal volume of Matrigel matrix gel and mixed well. Then, the mixture is inoculated onto a multi well plate and the plate is placed in a culture incubator for 30-60 minutes until Matrigel is completely coagulated. Then, the culture medium for hepatoma organoid is added for expansion and cultivation.

The invention also provides a method for evaluating or screening a drug for treating liver cancer, comprising the following steps:
(1) culturing hepatoma organoid by using the method for culturing hepatoma organoid of the invention;
(2) selecting the drug to be tested and diluting into required concentration gradients;
(3) adding the drug which has been diluted to the organoid obtained in step (1);
(4) detecting the size or the viability of the organoid.

The beneficial effects of the invention include:
(1) the success rate for culturing hepatoma organoid is improved, with a success rate of 90% or more;
(2) the hepatoma organoid primary cultured *in vitro* is ensured to maintain the pathological characteristics of the patient;
(3) the hepatoma organoid can be rapidly cultured with high expansion efficiency, and the expanded hepatoma organoid has the ability of continuous passage;
(4) the cost for culture is controllable, as the culture medium does not require expensive Wnt agonists, R-spondin family proteins, Noggin proteins, BMP inhibitors, fibroblast growth factor 10 (FGF10) and the like factors;
(5) this technology can culture and provide the hepatoma organoid with large quantity, which is suitable for high-throughput screening of candidate compounds and high-throughput drug sensitivity functional tests *in vitro* for patients.

### Description of Drawings

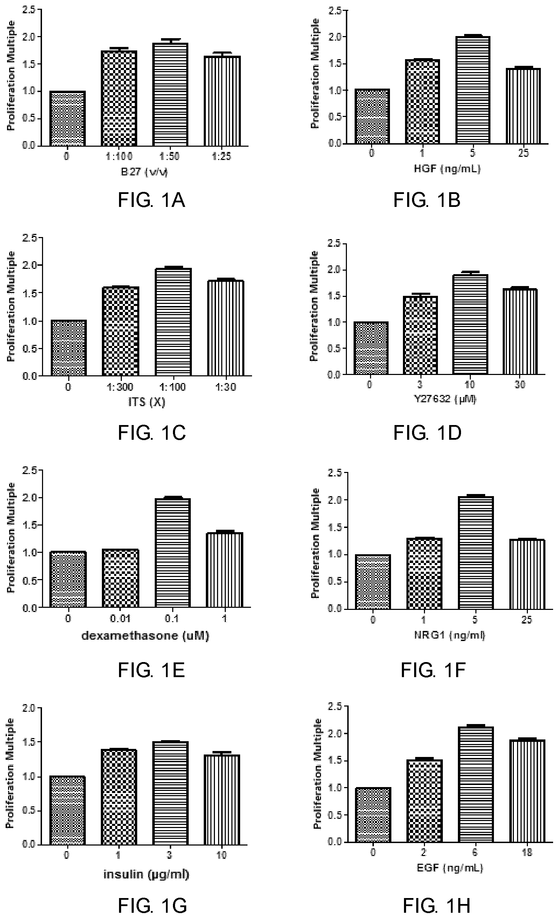

Figures 1A-1K show the effects of different concentrations of factors added to the culture medium for hepatoma organoid of the invention on the proliferation of hepatoma organoids.

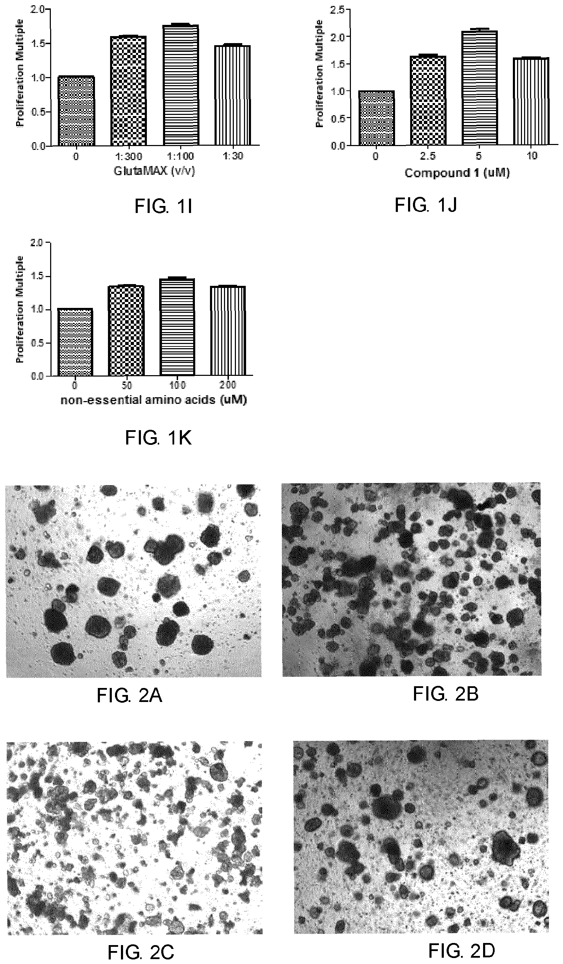

Figures 2A-2D are photos under a microscope of hepatoma organoids cultured using the culture medium for hepatoma organoid of the invention, wherein Figure 2A shows the photo of organoids cultured for 10 days from Sample GL-003; Figure 2B shows the photo of organoids cultured for 10 days from Sample GL-006; Figure 2C shows the photo of organoids cultured for 12 days from Sample GL-008; Figure 2D shows the photo of organoids cultured for 15 days from Sample GL-013.

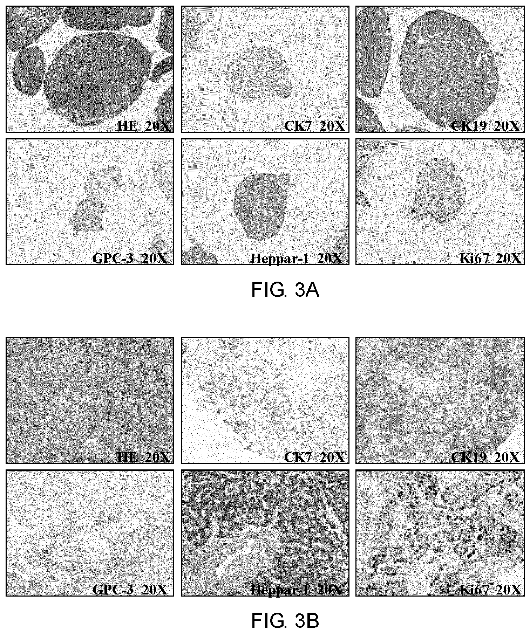

Figure 3A shows the pathological and immunohistochemical identification results of hepatoma organoids cultured from Sample GL-006 using the culture medium for hepatoma organoid of the invention; Figure 3B shows the pathological and immunohistochemical identification results of the tissue sample GL-006.

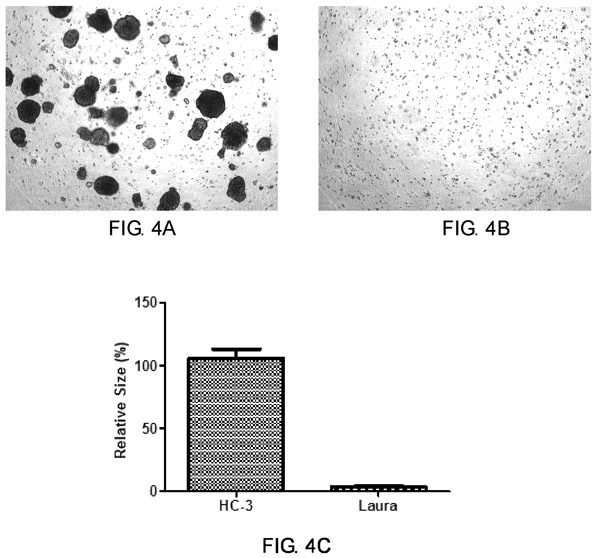

Figure 4 shows the comparison results of cultivation of hepatoma organoids between the culture medium for hepatoma organoid of the invention and the existing culture medium, wherein Figure 4A shows a photo of the organoids cultured for 25 days using the HC-3 culture medium of the invention; Figure 4B shows a photo of the organoids cultured for 25 days using Laura culture medium; Figure 4C shows a bar chart comparing the relative sizes of organoids cultured by HC-3 culture medium and Laura culture medium.

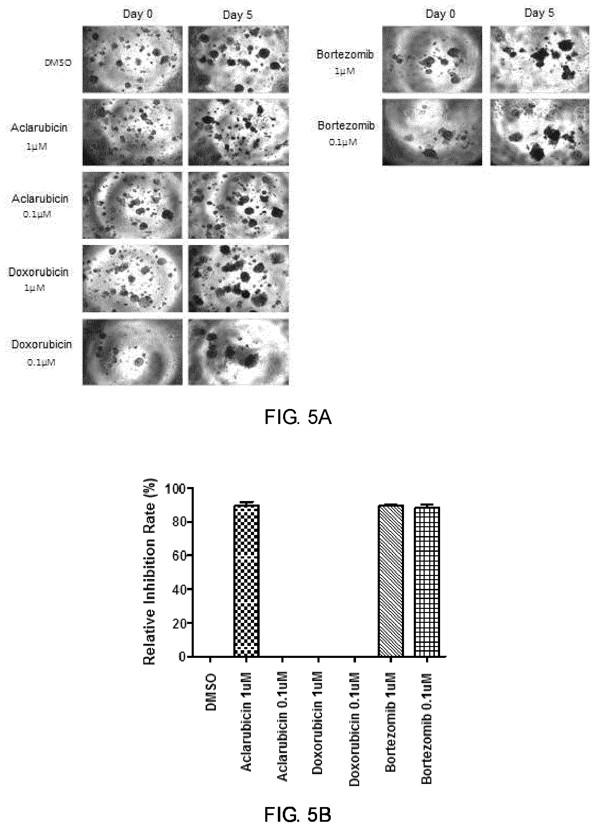

Figure 5 shows the results of different drug sensitivity tests on hepatoma organoids cultured by using the culture medium for hepatoma organoid of the invention, wherein Figure 5A shows photos of the growth of organoids without drug treatment and photos of the growth of organoids after 5 days of drug treatment; Figure 5B shows the bar chart of the inhibition rates on the growth of hepatoma organoids by different concentrations of the testing drugs.

### Detailed Description of the Invention

In order to better understand the invention, it is further described below in combination with the embodiments and the drawings. The following examples are provided only for the purpose of illustrating, but not for limiting the invention.

### [Preparation Examples of MST1/2 Kinase Inhibitors]

In the specification, MST1/2 kinase inhibitor refers to any inhibitor that directly or indirectly negatively regulates MST1/2 signaling. Generally, MST1/2 kinase inhibitors reduce the activity of MST1/2 kinase by, for example, binding to the same. Since MST1 and MST2 have similar structures, MST1/2 kinase inhibitors may be, for example, compounds that bind to MST1 or MST2 and reduce the activity thereof.

### 1. Preparation of MST1/2 kinase inhibitor Compound 1

### 4-((7-(2,6-Difluorophenyl)-5,8-dimethyl-6-oxo-5,6,7,8-tetrahydropteridin-2-yl) amino)benzsulfamide 1

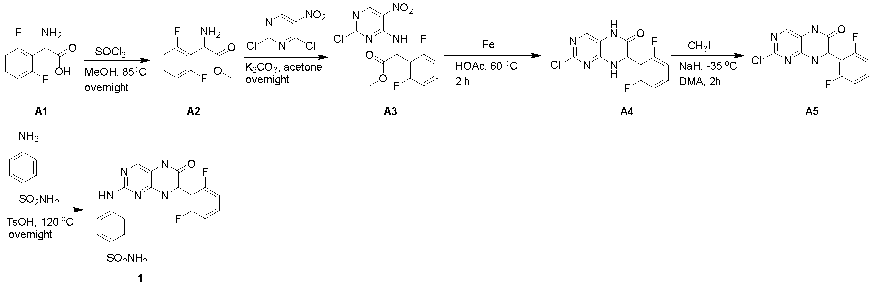

Methyl 2-amino-2-(2,6-difluorophenyl)acetate (A2): 2-amino-2-(2,6-difluorophenyl)acetic acid (2.0 g) and then methanol (30 ml) were added into a round bottom flask, followed by addition of thionyl chloride (1.2 ml) dropwise under an ice bath. The reaction system was reacted overnight at 85°C. After the completion of the reaction, the system was evaporated under reduced pressure to dry the solvent, and the obtained white solid was directly used in the next step.

Methyl 2-((2-chloro-5-nitropyrimidin-4-yl)amino)-2-(2,6-difluorophenyl) acetate (A3): methyl 2-amino-2-(2,6-difluorophenyl)acetate (2 g) and then acetone (30 ml) and potassium carbonate (2.2 g) were added into a round bottom flask, and then the system was cooled to -10°C with an ice salt bath, and then a solution of 2,4-dichloro-5-nitropyrimidine (3.1 g) in acetone was slowly added. The reaction system was stirred overnight at room temperature. After the completion of the reaction, the reaction mixture was filtered, the solvent was removed from the filtrate under reduced pressure, and the residue was purified by pressurized silica gel column chromatography to obtain compound A3. LC/MS: M+H 359.0.

2-Chloro-7-(2,6-difluorophenyl)-7,8-dihydropteridin-6(5H)-one (A4): methyl 2-((2-chloro-5-nitropyrimidin-4-yl)amino)-2-(2,6-difluorophenyl)acetate (2.5 g) and then acetic acid (50 ml) and iron powder (3.9 g) were added into a round bottom flask. The reaction system was stirred at 60°C for two hours. After the completion of the reaction, the reaction system was evaporated under reduced pressure to dry the solvent, and the resultant was neutralized to alkaline with saturated sodium bicarbonate solution and was extracted with ethyl acetate. The organic phase was washed with water and saturated brine and dried with anhydrous sodium sulfate. The organic phase was filtered and evaporated to dryness under reduced pressure to obtain a crude product. The crude product was washed with diethyl ether to obtain compound A4. LC/MS: M+H 297.0.

2-Chloro-7-(2,6-difluorophenyl)-5,8-dimethyl-7,8-dihydropteridin-6(5H)-one (A5): 2-chloro-7-(2,6-difluorophenyl)-7,8-dihydropteridin-6(5H)-one (2 g) and N,N-dimethylacetamide (10 ml) were added into a round bottom flask, and cooled to -35°C, followed by addition of iodomethane (0.9 ml) and then sodium hydride (615 mg), and the reaction system was stirred for two hours. After the completion of the reaction, the reaction mixture was quenched with water, and extracted with ethyl acetate. The organic phase was washed with water and saturated brine, respectively, and dried with anhydrous sodium sulfate. The organic phase was filtered and evaporated to dryness under reduced pressure to obtain a crude product. The crude product was washed with diethyl ether to obtain compound A5. LC/MS: M+H 325.0.

4-((7-(2,6-Difluorophenyl)-5,8-dimethyl-6-oxo-5,6,7,8-tetrahydropteridin-2-yl )amino)benzsulfamide (1): 2-chloro-7-(2,6-difluorophenyl)-5,8-dimethyl-7,8-dihydropteridin-6(5H)-one (100 mg), sulfanilamide (53 mg), p-toluenesulfonic acid (53 mg) and sec-butanol (5 ml) were added into a round bottom flask. The reaction system was stirred at 120°C overnight. After the completion of the reaction, the reaction mixture was filtered, and washed with methanol and diethyl ether to obtain compound 1. LC/MS: M+H 461.1.

### 2. Preparation of other MST1/2 inhibitor compounds of the invention

Other MST1/2 inhibitor compounds of the invention were synthesized via the method similar to that of Compound 1, and their structures and mass spectrum data are shown in the following table.

| No. | Compound | MS(ESI) m/z(M+1)+ | No. | Compound | MS(ESI) m/z(M+1)+ |
|---|---|---|---|---|---|
| 1 | 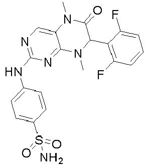 | 461.1 | 2 | 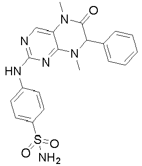 | 425.14 |
| 3 | 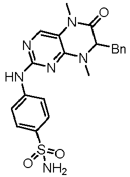 | 439.15 | 4 | 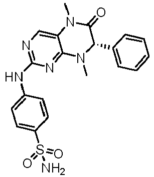 | 425.14 |
| 5 | 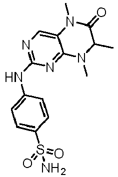 | 363.12 | 6 | 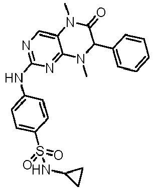 | 465.17 |
| 7 | 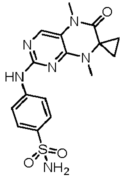 | 375.12 | 8 | 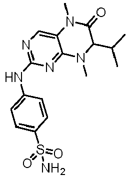 | 391.16 |
| 9 | 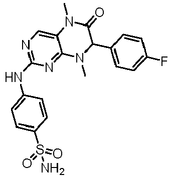 | 443.13 | 10 | 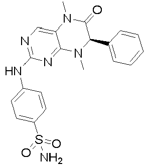 | 425.14 |
| 11 | 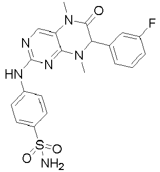 | 443.13 | 12 | 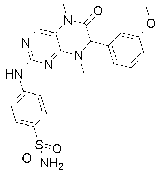 | 455.15 |
| 13 | 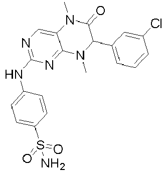 | 459.10 | 14 | 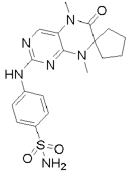 | 403.15 |
| 15 | 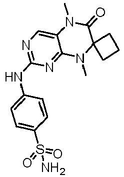 | 389.14 | 16 | 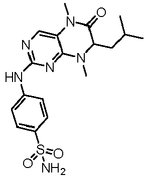 | 405.17 |
| 17 | 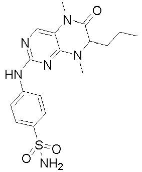 | 391.15 | 18 | 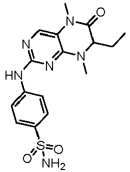 | 377.14 |
| 19 | 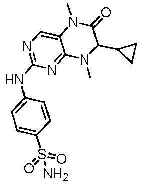 | 389.14 | 20 | 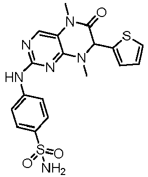 | 431.09 |
| 21 | 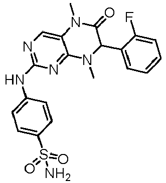 | 443.13 | 22 | 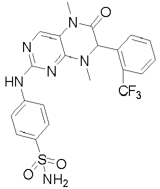 | 493.12 |
| 23 | 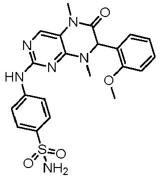 | 455.15 | 24 | 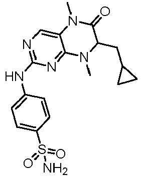 | 403.15 |
| 25 | 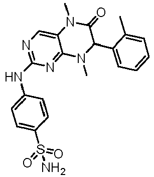 | 439.15 | 26 | 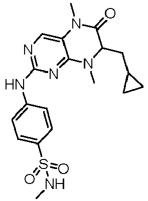 | 417.17 |
| 27 | 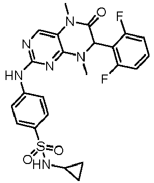 | 501.15 | 28 | 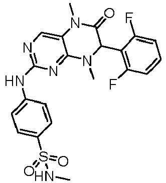 | 475.13 |
| 29 | 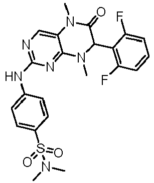 | 489.15 | 30 | 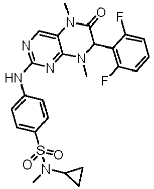 | 515.16 |
| 31 | 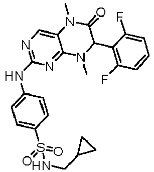 | 515.16 | 32 | 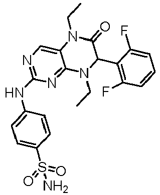 | 489.15 |
| 33 | 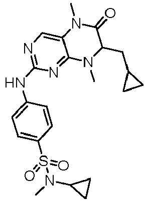 | 457.20 | 34 | 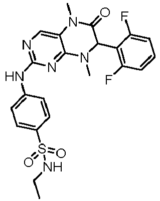 | 489.15 |
| 35 | 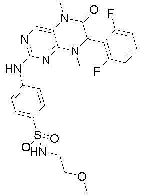 | 519.16 | 36 | 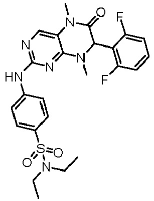 | 517.18 |
| 37 | 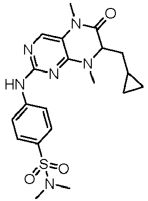 | 431.18 | 38 | 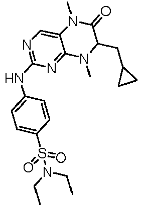 | 459.21 |
| 39 | 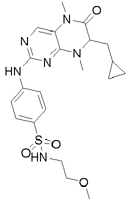 | 461.19 | 40 | 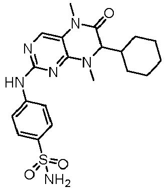 | 431.19 |
| 41 | 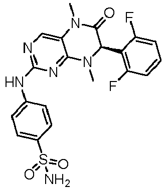 | 461.12 | 42 | 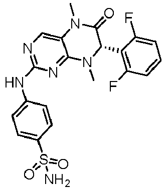 | 461.12 |
| 43 | 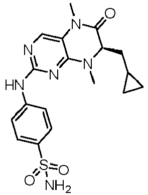 | 403.15 | 44 | 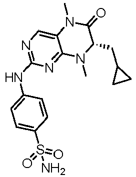 | 403.15 |
| 45 | 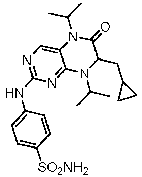 | 459.21 | 46 | 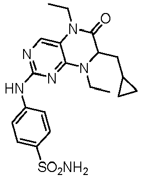 | 431.18 |
| 47 | 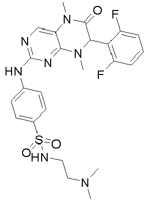 | 532.19 | 48 | 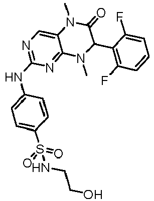 | 505.14 |
| 49 | 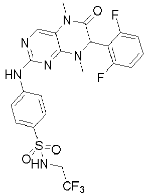 | 543.12 | 50 | 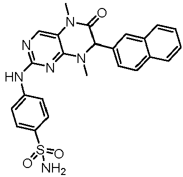 | 475.16 |
| 51 | 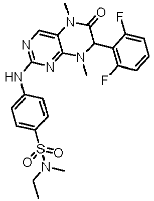 | 503.17 | 52 | 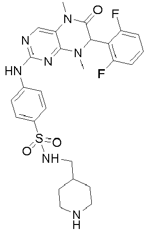 | 558.21 |
| 53 | 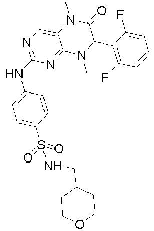 | 559.19 | 54 | 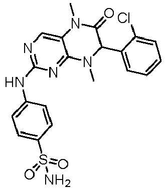 | 459.10 |
| 55 | 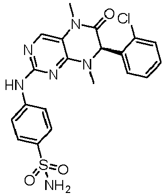 | 459.10 | 56 | 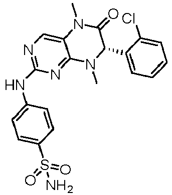 | 459.10 |
| 57 | 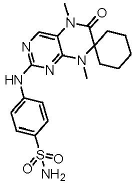 | 417.17 | 58 | 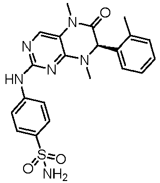 | 439.16 |
| 59 | 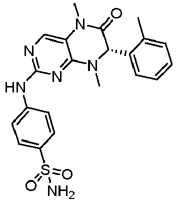 | 439.16 | | | |

### Example 1. The effects of various factors added to culture medium for hepatoma organoid on the proliferation of hepatoma organoids

### (1) Preparation of culture medium for hepatoma organoids

First, a basic medium containing initial medium was prepared. The initial medium can be selected from commonly used DMEM/F12, DMEM, F12, or RPMI-1640. In this embodiment, the formulation of the basic medium is: DMEM/F12 medium (purchased from Corning) + 100 µg/mL Primocin (purchased from InvivoGen, 0.2% (v/v), commercial product has a concentration of 50 mg/ml).

Different types of additives (see Table 1) were added to the basic medium to prepare culture mediums for hepatoma organoid with different components.

### (2) Isolation and treatment of primary liver cancer cells

### 1. Sample Selection

Tissue samples (intraoperative) of liver cancer solid tumor were obtained from patients by professional medical practitioner of professional medical institutions, and all patients have signed the informed consent. Intraoperative samples having the size of 0.25 cm³ were stored and transported using commercial tissue preservation solution (manufacturer: Miltenyi Biotec).

### 2. Material preparation

After subjecting to surface sterilization, 15 mL sterile centrifuge tubes, pipettors, 10 mL pipettes, sterile pipette tips, etc., were put in an ultra-clean workbench for ultraviolet irradiation of 30 minutes. The basic medium was taken out from a 4°C refrigerator 30 minutes in advance, and the tissue digestion solution was taken out from a -20°C refrigerator 30 minutes in advance.

Tissue digestion solution: 1640 medium (Corning, 10-040-CVR), collagenase II (2 mg/mL), collagenase IV (2 mg/mL), DNase (50 U/mL), hyaluronidase (0.75 mg/mL), calcium chloride (3.3 mM), BSA (10 mg/mL).

Collagenase II, collagenase IV, DNase, and hyaluronidase mentioned above were all purchased from Sigma Corporation; calcium chloride was purchased from Sangon Biotech (Shanghai) Co., Ltd.; BSA was purchased from Biofroxx Corporation.

### 3. Sample isolation

3.1. Tissue samples were transferred from the ultra-clean workbench to a culture dish, and the tissue with blood was removed. The tissue samples were rinsed twice with basic medium, and then were transferred to another culture dish and mechanically cut with a sterile scalpel into tissue blocks of 1×1×1 mm³ in size.

3.2. The cut intraoperative tissues were aspirated into a 15 mL centrifuge tube, to which 5 mL of basic medium was added, fully mixed, and then centrifuged at 1500 rpm for 4 minutes.

3.3. The supernatant was discarded, and the resultant was added with 1:3 mixture of basic medium and tissue digestion solution (the amount of tissue digestion solution added was about 10 mL tissue digestion solution for 1 g of tumor tissue). The samples were marked with names and numbers, sealed with sealing film, and then digested in a shaker (Zhichu Instrument ZQLY-180N) at 37°C, 300 rpm. The completion of digestion was determined via observation every 30 minutes, based on the existing of visible particles or not.

3.4. After the digestion was completed, undigested tissue mass was filtered out through a 100 µM filter mash. The tissue mass on the filter mash was rinsed into the centrifuge tube with basic medium to reduce cell loss. The resultant was centrifuged at 25°C, 1500 rpm for 4 minutes.

3.5. The supernatant was discarded and the resultant was observed to determine whether there were blood cells. If there were blood cells, 8 mL blood cell lysate (purchased from Sigma) was added in the resultant and fully mixed, lysed at 4°C for 20 minutes, with inverting and mixing once during the process. The resultant was centrifuged at 25°C, 1500 rpm for 4 minutes.

3.6. The supernatant was discarded and 2 mL basic medium was added to resuspend the cells for reserve.

### 4. Cell count and treatment

4.1 Microscopic observation: a small amount of resuspended cells were plated in a culture dish, and the density and morphology of cancer cells were observed under a microscope (CNOPTEC, BDS400);
4.2 Viable cell counting: 12 µL of the resuspended cell suspension was fully mixed with 12 µL of trypan blue staining solution (Manufacturer: Sangon Biotech (Shanghai) Co., Ltd.), and then 20 µL of the mixture was added into a cell counting plate (Manufacturer: Countstar, specifications: 50 pieces/box). The percentage of viable large cells (cell size > 10 µm) = number of viable cells / number of total cells × 100%, was calculated with a cell counter (Countstar, IC1000).

### (3) Culture of hepatoma organoids

The primary liver cancer cells obtained in the above steps were resuspended with pre-cooled DMEM/F12 and counted. The cell density was diluted to 5-10×10⁶ cells/mL and 400 µL of the diluted cell suspension was added with an equal volume of Matrigel matrix gel (Corning) and mixed gently and well. Then, the mixture was inoculated at 5 µL/well onto a 96-well plate. The inoculated plate was placed into an incubator for 30 minutes until Matrigel is completely coagulated. Then, the culture mediums as shown in Table 1 that have been come to room temperature in advance were added, respectively, and the culture mediums were renewed every three days for expansion and cultivation. After 7 days, the cultured organoids were photographed and the diameters of the organoids were measured and compiled, so as to compare the promoting effects of various factors on the proliferation of hepatoma organoids. As the experimental control, the basic medium without any additives was used. The experimental results are shown in Table 1.

**Table 1: Additives in the culture mediums and their effects on promoting organoid proliferation**

| No. | Types of medium additive | Supplier | Final concentration | Level of promoting proliferation |
|---|---|---|---|---|
| 1 | N2 | Gibco | 1:50 | + |
| 2 | epidermal growth factor, EGF | R&D | 5ng/mL | + |
| 3 | R-spondin1 | R&D | 20ng/mL | + |
| 4 | prostaglandin E2 | Tocris | 0.5µM | ○ |
| 5 | insulin | Peprotech | 1.5µg/mL | + |
| 6 | B27 | Gibco | 1:50 | + |
| 7 | A8301 | MCE | 100nM | - |
| 8 | SB202190 | MCE | 200nM | - |
| 9 | basic fibroblast growth factor, bFGF | R&D | 10ng/mL | - |
| 10 | hydrocortisone | Sigma | 10ng/mL | ○ |
| 11 | Noggin | R&D | 30ng/mL | ○ |
| 12 | fetal bovine serum, FBS | Excell | 5% | + |
| 13 | insulin-like growth factor-1, IGF-1 | R&D | 45ng/mL | ○ |
| 14 | keratinocyte growth factor, KGF | R&D | 5ng/mL | ○ |
| 15 | GlutaMAX | Gibco | 1:100 | + |
| 16 | non-essential amino acids | Corning | 100µM | + |
| 17 | dexamethasone | MCE | 0.1µM | + |
| 18 | Neuregulin-1, NRG1 | sino biological | 5ng/mL | + |
| 19 | Y27632 | MCE | 10µM) | + |
| 20 | ITS cell culture additive | Gibco | 1:100 | + |
| 21 | Compound 1 | Preparation Example | 5µM | + |
| 22 | CHIR99021 | MCE | 2.5µM | - |
| 23 | hepatocyte growth factor, HGF | R&D | 5ng/mL | + |

Wherein, "+" indicates that compared with the Basic Medium, the medium added with the additive(s) has the effect of promoting the proliferation of hepatoma organoids isolated from liver cancer tissue in at least two cases; "-" indicates that the medium added with the additive(s) has the effect of inhibiting the proliferation of hepatoma organoids isolated from liver cancer tissue in at least one case; "o" indicates that the medium added with the additive(s) has no significant effect on the proliferation of hepatoma organoids isolated from liver cancer tissue in at least two cases.

According to the above results, factors including B27, hepatocyte growth factor (HGF), ITS cell culture additive, Y27632, dexamethasone, Neuregulin-1 (NRG1), insulin, epidermal growth factor (EGF), GlutaMAX, Compound 1, and non-essential amino acids were selected for further experiments of culture.

### Example 2. Effects of different concentrations of factors added in the culture medium on the proliferation of hepatoma organoids

Primary liver cancer cells were obtained from intraoperative tissue samples (Nos. GL-003, GL-004) in accordance with the method described in Section (2), Example 1, and the organoids were cultured using the culture medium formulations as shown in Table 2 below.

**Table 2: Culture medium formulations (final concentrations are shown)**

| Culture Medium | Component |
|---|---|
| Basic Medium (BM) | DMEM/F12 +100 µg/mL Primocin |
| HC-3 medium | BM + 1:50 B27 additive + 5ng/mL HGF + 1:100 ITS cell culture additive + 10µM Y27632 + 0.1µM dexamethasone + 5ng/mL NRG1 + 3µg/mL insulin + 6ng/mL EGF + 1: 100 GlutaMAX + 5µM Compound 1 + 100µM non-essential amino acids |
| Formulation 1 | HC-3 medium - 1:50 B27 additive |
| Formulation 2 | HC-3 medium - 5ng/mL HGF |
| Formulation 3 | HC-3 medium - 1: 100 ITS cell culture additive |
| Formulation 4 | HC-3 medium - 10µM Y27632 |
| Formulation 5 | HC-3 medium - 0.1µM dexamethasone |
| Formulation 6 | HC-3 medium - 5ng/mL NRG1 |
| Formulation 7 | HC-3 medium - 3µg/mL insulin |
| Formulation 8 | HC-3 medium - 6ng/mL EGF |
| Formulation 9 | HC-3 medium - 1:100 GlutaMAX |
| Formulation 10 | HC-3 medium - 5µM Compound 1 |
| Formulation 11 | HC-3 medium - 100µM non-essential amino acids |

When using the culture medium of Formulation 1, to a 96-well plate inoculated with organoids was added the prepared B27 in addition to the medium of Formulation 1, with 200 µL per well, such that the final concentrations of B27 were 1:25, 1:50 and 1:100, respectively; a well of Blank Control (BC) was set using the medium of Formulation 1. The final concentrations of other factors added in this series of culture mediums were the same as those in HC-3 culture medium. The experiments of Formulations 1-11 below were also conducted in the same way and will not be described in details.

When using the culture medium of Formulation 2, to a 96-well plate inoculated with organoids was added the prepared HGF in addition to the medium of Formulation 2, with 200 µL per well, such that the final concentrations of HGF were 1 ng/mL, 5 ng/mL and 25 ng/mL, respectively; a well of Blank Control (BC) was set using the medium of Formulation 2.

When using the culture medium of Formulation 3, to a 96-well plate inoculated with organoids was added the prepared ITS cell culture additive in addition to the medium of Formulation 3, with 200 µL per well, such that the final concentrations of ITS cell culture additive were 1:300, 1:100 and 1:30, respectively; a well of Blank Control (BC) was set using the medium of Formulation 3.

When using the culture medium of Formulation 4, to a 96-well plate inoculated with organoids was added the prepared Y27632 in addition to the medium of Formulation 4, with 200 µL per well, such that the final concentrations of Y27632 were 3 µM, 10 µM and 30 µM, respectively; a well of Blank Control (BC) was set using the medium of Formulation 4.

When using the culture medium of Formulation 5, to a 96-well plate inoculated with organoids was added the prepared dexamethasone in addition to the medium of Formulation 5, with 200 µL per well, such that the final concentrations of dexamethasone were 0.01 µM, 0.1 µM and 1 µM, respectively; a well of Blank Control (BC) was set using the medium of Formulation 5.

When using the culture medium of Formulation 6, to a 96-well plate inoculated with organoids was added the prepared NRG1 in addition to the medium of Formulation 6, with 200 µL per well, such that the final concentrations of NRG1 were 1 ng/mL, 5 ng/mL and 25 ng/mL, respectively; a well of Blank Control (BC) was set using the medium of Formulation 6.

When using the culture medium of Formulation 7, to a 96-well plate inoculated with organoids was added the prepared insulin in addition to the medium of Formulation 7, with 200 µL per well, such that the final concentrations of insulin were 1 µg/mL, 3 µg/mL and 10 µg/mL, respectively; a well of Blank Control (BC) was set using the medium of Formulation 7.

When using the culture medium of Formulation 8, to a 96-well plate inoculated with organoids was added the prepared EGF in addition to the medium of Formulation 8, with 200 µL per well, such that the final concentrations of EGF were 2 ng/mL, 6 ng/mL and 18 ng/mL, respectively; a well of Blank Control (BC) was set using the medium of Formulation 8.

When using the culture medium of Formulation 9, to a 96-well plate inoculated with organoids was added the prepared GlutaMAX in addition to the medium of Formulation 9, with 200 µL per well, such that the final concentrations of GlutaMAX were 1:300, 1:100 and 1:30, respectively; a well of Blank Control (BC) was set using the medium of Formulation 9.

When using the culture medium of Formulation 10, to a 96-well plate inoculated with organoids was added the prepared Compound 1 in addition to the medium of Formulation 10, with 200 µL per well, such that the final concentrations of Compound 1 were 2.5 µM, 5 µM and 10 µM, respectively; a well of Blank Control (BC) was set using the medium of Formulation 10.

When using the culture medium of Formulation 11, to a 96-well plate inoculated with organoids was added the prepared non-essential amino acids in addition to the medium of Formulation 11, with 200 µL per well, such that the final concentrations of non-essential amino acids were 50 µM, 100 µM and 200 µM, respectively; a well of Blank Control (BC) was set using the medium of Formulation 11.

After 10 days, the cultured organoids were photographed and the diameters of the organoids were measured and compiled, so as to compare the promoting effects of various factor concentrations on the proliferation of hepatoma organoids. The data collected from two samples were summarized and shown in Figures 1Ato 1K. In Figures 1Ato 1K, the ratio represents a ratio of the diameter of organoids cultured by using each culture medium for 10 days to the diameter of organoids cultured by using the corresponding culture medium in the BC well for 10 days. A ratio greater than 1 indicates that the culture medium containing different concentrations of factors or small molecule compounds has a better proliferation promoting effect than the culture medium in the well of Blank Control; a ratio less than 1 indicates that the culture medium containing different concentrations of factors or small molecule compounds has a poorer proliferation promoting effect than the culture medium in the well of Blank Control.

According to the results of Figures 1A to 1K, the volume concentration of B27 is preferably 1:25 to 1:100; the amount of hepatocyte growth factor is preferably 1-25 ng/mL; the volume concentration of ITS cell culture additive is preferably 1:30 to 1:300; the amount of Y27632 is preferably 3-30 µM; the amount of dexamethasone is preferably 0.1-1 µM; the amount of NRG1 preferably is 1-25 ng/mL; the amount of insulin is 1-10 µg/mL; the amount of epidermal growth factor is preferably 2-18 ng/mL; the volume concentration of GlutaMAX is preferably 1:30 to 1:300; the amount of MST1/2 kinase inhibitor Compound 1 is preferably 2.5-10 µM; the amount of non-essential amino acids is preferably 50-200 µM.

### Example 3. Culture and identification of hepatoma organoids

The primary liver cancer cells (GL-003, GL-006, GL-008, GL-013) obtained by the method described in Section (2), Example 1 were resuspended using the HC-3 culture medium for hepatoma organoid of the invention and counted. The cell density was diluted to 5-10×10⁶ cells/mL and 400 µL of the diluted cell suspension was added with an equal volume of Matrigel matrix gel (Corning) and mixed gently and well. Then, the mixture was inoculated at 50 µL/well onto a 24-well plate. The inoculated plate was placed into an incubator for 30 minutes until Matrigel is completely coagulated. Then, the HC-3 culture medium for hepatoma organoid that has been come to room temperature in advance were added, with 500 µL per well, and the culture medium were renewed every three days for expansion and cultivation.

On Day 10-15, the cultured hepatoma organoids were observed using a microscope (Invitrogen, EVOS M500). Figures 2A-2D show photos of hepatoma organoids cultured from Samples GL-003 (Day 10), GL-006 (Day 10), GL-008 (Day 12), and GL-013 (Day 15) taken under a 4x objective lens. Hepatoma organoids appear in regular spherical shapes, with relatively smooth surfaces, under the microscope.

Pathological and immunohistochemical identification was performed on the cultured hepatoma organoids, and the corresponding tissue samples were also sent for pathological and immunohistochemical identification to compare the consistency of results between the organoids and tissues.

Figure 3A shows the pathological and immunohistochemical identification results of hepatoma organoids cultured *in vitro* from Sample GL-006, which are photos taken under a 20x objective lens, respectively. As shown in Figure 3A, the HE result shows that the structural morphology of organoids is cancer tissue morphology; CK19, Heppar-1, and Ki67 are expressed, indicating that the sample is liver cancer. Figure 3B shows the pathological and immunohistochemical results of the corresponding tissue of GL-006 before cultivation, indicating that the diagnostic results of hepatoma organoids cultured using the HC-3 culture medium of the invention are consistent with those of liver cancer tissue before cultivation.

### Example 4. Comparison of the culture effect with that of the existing culture medium

### (1) Preparation of control culture medium

Culture medium used in a literature (Laura et al., Nat Med. 2017, 23(12): 1424-1435) was prepared, which has the formulation of: Advanced DMEM/F12 medium (purchased from Corning) + 1:100 Penicillin/Streptomycin (purchased from Corning) + 1:100 GlutaMAX (purchased from Corning) + 10 mM HEPES (purchased from Thermo Fisher) +1:50 B27 (purchased from Gibco) + 1:100 N2 (purchased from Gibco) + 1.25 mmol/L N-acetylcysteine (purchased from MCE) +10 mmol/L nicotinamide (purchased from MCE) + 10 nM gastrin (purchased from MCE) + 50 ng/ml epidermal growth factor (purchased from R&D) + 100 ng/ml fibroblast growth factor 10 (purchased from sino biological) + 25 ng/ml hepatocyte growth factor (purchased from R&D) + 10 µmol/L Forskolin (purchased from MCE)+ 5 µmol/L A8301 (purchased from MCE) + 10 µmol/L Y27632 (purchased from MCE) + 3 nmol/L dexamethasone (purchased from MCE). Hereinafter, it referred to as Laura culture medium.

### (2) Culture of hepatoma organoids

Primary liver cancer cells were obtained from intraoperative tissue sample GL-018 according to the method described in Section (2), Example 1, and were cultured to obtain organoids by using HC-3 culture medium and Laura culture medium, respectively, according to the method described in Example 3.

On Day 25 of cultivation, the cultured hepatoma organoids were observed using a microscope (Invitrogen, EVOS M500). Figures 4A and 4B are photos taken under a 4x objective lens of the organoids cultured in HC-3 culture medium and Laura culture medium, respectively. Figure 4C is a bar chart comparing the relative sizes of organoids cultured using the two culture mediums.

According to the results of Figures 4A-4C, it can be seen that compared with Laura culture medium, HC-3 culture medium can significantly promote the expansion and cultivation of hepatoma organoids.

Example 5. Drug screening using the hepatoma organoids obtained by expending with the culture medium of the invention

### (1) Culture of hepatoma organoids

Primary liver cancer cells were isolated from liver cancer intraoperative sample (GL-006) according to the method described in Section (2), Example 1, and were cultured to obtain organoids by using HC-3 culture medium until the diameters of hepatoma organoids exceeded 50 µm, which were then used for drug screening.

### (2) Preparation of drugs for screening

Prepare Three drugs with two concentration gradients were prepared according to the table below (Bortezomib, Aclarubicin, and Doxorubicin; all purchased from MCE) and store for use.

**Table 3: Preparation of Drug Additions of Aclarubicin, Doxorubicin, and Bortezomib**

| | | | |
|---|---|---|---|
| Aclarubicin | Preparation concentration (mM) | 1 | 0.1 |
| Doxorubicin | Preparation concentration (mM) | 1 | 0.1 |
| Bortezomib | Preparation concentration (mM) | 1 | 0.1 |

### (3) Drug loading

The prepared drugs were taken out and placed at room temperature. The drugs were diluted 1000 times with HC-3 culture medium for later use. The organoids cultured and obtained according to step (1) were taken out from the incubator, the culture mediums were removed from the wells, and the culture mediums containing drugs were slowly added into the wells along the well wall. After the addition of drug, disinfect the surface of the 96-well plate was sterilized and then was transferred to the incubator for further cultivation. After 5 days, the viability of organoids were measured.

### (4) Detecting of viability of organoids

CellTiter-Glo luminescent reagent (purchased from Promega) was taken out from a 4°C refrigerator, and 10 mL of the reagent was added into the loading slot; the 96-well plate for testing was taken out from the incubator, and 20 µL CellTiter-Glo luminescent reagent was added into each well. After standing for 10 minutes followed by mixing well, the test was conducted by using a multi-functional microplate reader (Envision, Perkin Elmer).

### (5) Data processing

According to the formula, Drug inhibition rate (%) = 100% - (Chemiluminescence value of drug-treatment well on Day 5 / Chemiluminescence value of drug-treatment well on Day 0) / (Chemiluminescence value of DMSO well on Day 5 / Chemiluminescence value of DMSO well on Day 0) × 100%, the inhibition rates of different drugs were calculated. The results were shown in Figures 5A and 5B. Figure 5A shows photos of the growth of organoids without drug treatment and photos of the growth of organoids after 5 days of drug treatment, which were taken under a 4X objective microscope (Invitrogen, EVOS M500). Figure 5B shows the bar charts of the inhibition rates on the growth of hepatoma organoids by different concentrations of the testing drugs.

From Figure 5A, it can be confirmed that the growth of organoids cultured using the culture medium for hepatoma organoid of the invention was good, and the growth of organoids were significantly inhibited after the treatment with Bortezomib and Aclarubicin. Figure 5B shows the bar charts of the inhibition rates on the growth of hepatoma organoids by different concentrations of three testing drugs. From Figure 5B, it can be seen that the data errors of the drug treatment group were quite small, indicating that when using this system for drug screening, the data among the replicated wells of the same drug are substantially consistent. Among the three anti-tumor drugs, Bortezomib had strong inhibitory effects on the growth of organoids at two concentrations, while the inhibitory effects of Aclarubicin at different concentrations varied significantly, and Doxorubicin had no inhibitory effect on the growth of this hepatoma organoid strain. This indicated that the efficacy and sensitivity of organoids from the same patient to different drugs are different. Based on the results, the efficacy and the effective dosage of the drug used in the clinical for a liver cancer patient can be determined.

### Industrial Applicability

The invention provides a culture medium and a culture method for hepatoma organoid culture, and the cultured organoids can be used in efficacy evaluating and screening of drugs. Thus, the invention is suitable for industrial applications.

Although the invention has been described in detail herein, the invention is not limited thereto, and those skilled in the art can make modifications according to the principle of the invention. Therefore, any modification made in accordance with the principle of the invention shall be understood as falling within the protection scope of the invention.

## Claims

1. A culture medium for hepatoma organoid, **characterized in that**:
comprising an MST1/2 kinase inhibitor, at least one cell culture additive selected from N2 and B27, a hepatocyte growth factor, an ITS cell culture additive, Y27632, dexamethasone, Neuregulin-1, insulin, an epidermal cell growth factor, GlutaMAX, and non-essential amino acids,
wherein the MST1/2 kinase inhibitor comprises a compound of Formula (I) or a pharmaceutically acceptable salt, or a solvate thereof, 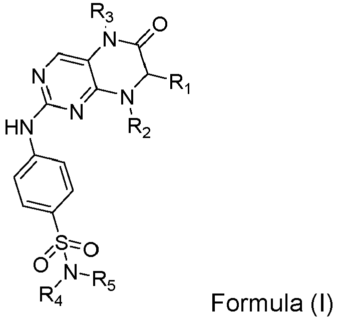 wherein,
R₁ is selected from C1-C6 alkyl, C3-C6 cycloalkyl, C4-C8 cycloalkylalkyl, C2-C6 spirocycloalkyl, and aryl optionally substituted with 1-2 independent R₆, aryl C1-C6 alkyl optionally substituted with 1-2 independent R₆ and heteroaryl optionally substituted with 1-2 independent R₆;
R₂ and R₃ are each independently selected from C1-C6 alkyl;
R₄ and R₅ are each independently selected from hydrogen, C1-C6 alkyl, C3-C6 cycloalkyl, C4-C8 cycloalkylalkyl, hydroxyl C1-C6 alkyl, C1-C6 haloalkyl, C1-C6 alkylamino C1-C6 alkyl, C1-C6 alkoxy C1-C6 alkyl, and C3-C6 heterocyclyl C1-C6 alkyl;
R₆ is selected from halogen, C1-C6 alkyl, C1-C6 alkoxy, and C1-C6 haloalkyl.

2. The culture medium of claim 1, wherein,
R₁ is selected from C1-C6 alkyl, C3-C6 cycloalkyl, C4-C8 cycloalkylalkyl, C2-C6 spirocycloalkyl, and phenyl optionally substituted with 1-2 independent R₆, naphthyl optionally substituted with 1-2 independent R₆, phenylmethyl optionally substituted with 1-2 independent R₆ and thienyl optionally substituted with 1-2 independent R₆;
R₂ and R₃ are each independently selected from C1-C3 alkyl;
R₄ and R₅ are each independently selected from hydrogen, C1-C6 alkyl, C3-C6 cycloalkyl, C4-C8 cycloalkylalkyl, hydroxyl C1-C6 alkyl, C1-C6 haloalkyl, C1-C6 alkylamino C1-C6 alkyl, C1-C6 alkoxy C1-C6 alkyl, piperidyl C1-C6 alkyl, and tetrahydropyranyl C1-C6 alkyl;
R₆ is selected from halogen, C1-C6 alkyl, C1-C6 alkoxy, and C1-C6 haloalkyl.

3. The culture medium of claim 1, wherein the MST1/2 kinase inhibitor comprises a compound of Formula (Ia) or a pharmaceutically acceptable salt, or a solvate thereof, 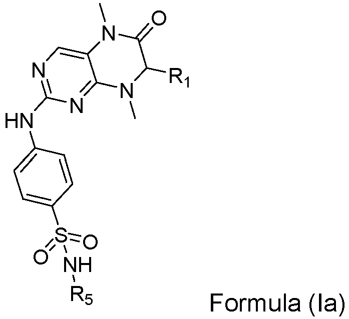 wherein,
R₁ is selected from C1-C6 alkyl, phenyl optionally substituted with 1-2 independent R₆, thienyl optionally substituted with 1-2 independent R₆, and phenylmethyl optionally substituted with 1-2 independent R₆;
R₅ is selected from hydrogen, C1-C6 alkyl, and C3-C6 cycloalkyl;
R₆ is independently selected from halogen, C1-C6 alkyl, and C1-C6 haloalkyl.

4. The culture medium of claim 3, wherein
R₁ is phenyl optionally substituted with 1-2 independent R₆;
R₅ is hydrogen;
R₆ is fluoro, methyl or trifluoromethyl.

5. The culture medium of claim 1, wherein the MST1/2 kinase inhibitor is at least one selected from the following compounds or a pharmaceutically acceptable salt thereof:
| | | | |
|---|---|---|---|
| 1 | 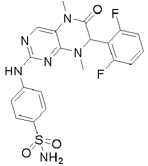 | 2 | 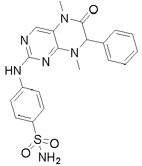 |
| 3 | 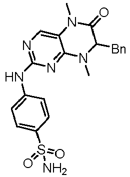 | 4 | 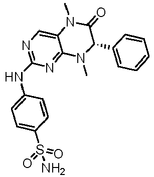 |
| 5 | 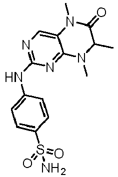 | 6 | 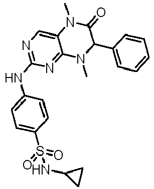 |
| 7 | 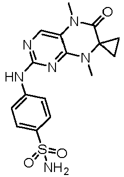 | 8 | 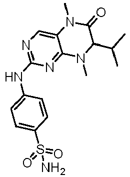 |
| 9 | 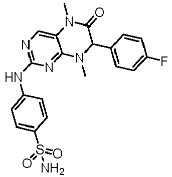 | 10 | 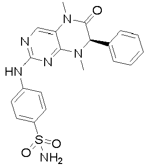 |
| 11 | 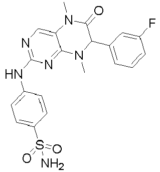 | 12 | 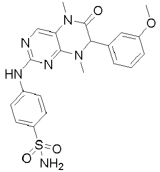 |
| 13 | 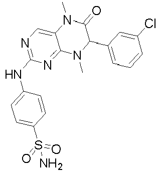 | 14 | 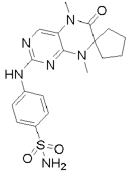 |
| 15 | 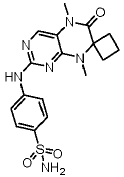 | 16 | 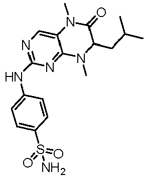 |
| 17 | 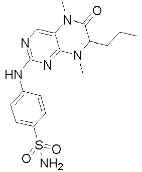 | 18 | 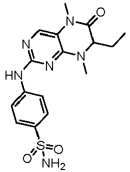 |
| 19 | 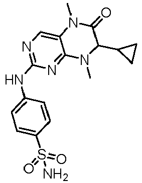 | 20 | 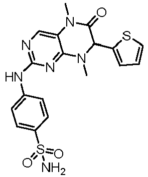 |
| 21 | 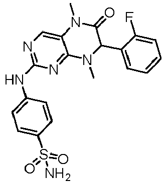 | 22 | 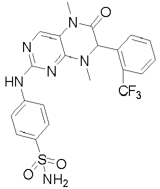 |
| 23 | 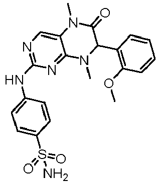 | 24 | 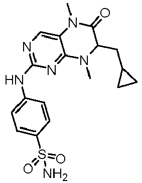 |
| 25 | 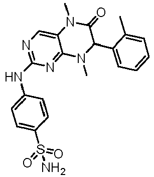 | 26 | 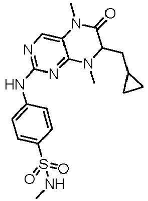 |
| 27 | 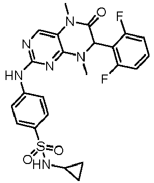 | 28 | 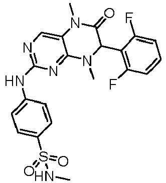 |
| 29 | 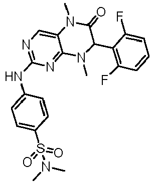 | 30 | 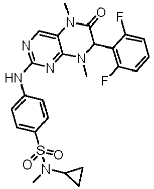 |
| 31 | 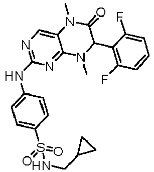 | 32 | 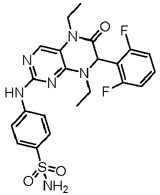 |
| 33 | 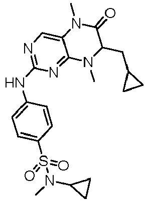 | 34 | 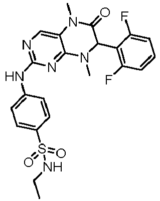 |
| 35 | 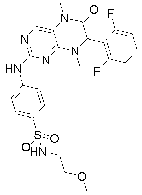 | 36 | 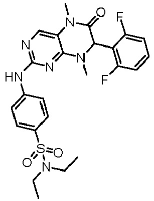 |
| 37 | 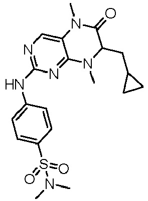 | 38 | 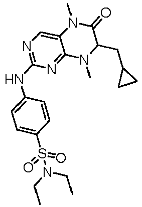 |
| 39 | 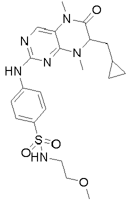 | 40 | 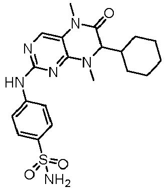 |
| 41 | 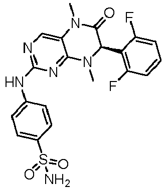 | 42 | 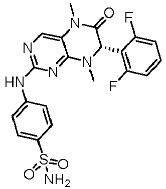 |
| 43 | 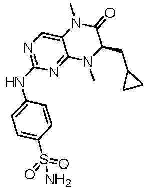 | 44 | 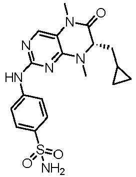 |
| 45 | 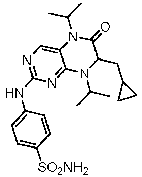 | 46 | 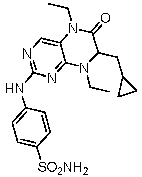 |
| 47 | 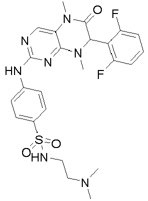 | 48 | 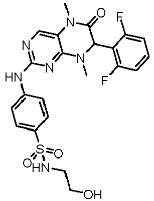 |
| 49 | 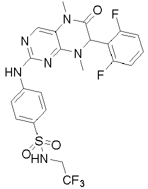 | 50 | 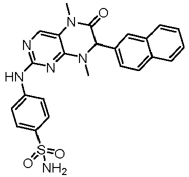 |
| 51 | 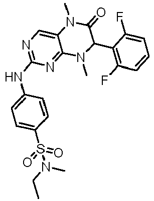 | 52 | 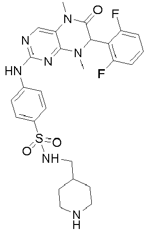 |
| 53 | 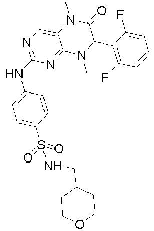 | 54 | 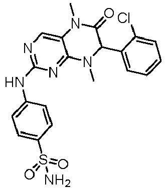 |
| 55 | 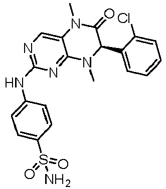 | 56 | 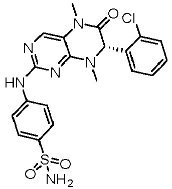 |
| 57 | 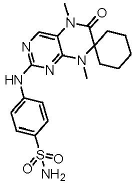 | 58 | 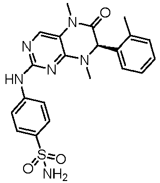 |
| 59 | 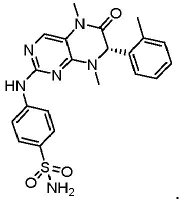 | | |

6. The culture medium of any one of claims 1-5, **characterized in that**, the amounts of the components in the culture medium satisfies any one or more or all of the following conditions:
the concentration of the MST1/2 kinase inhibitor is 2.5-10 µM);
the volume ratio of the B27 or N2 cell culture additive in the culture medium is 1:25-1:100;
the concentration of the hepatocyte growth factor is 1-25 ng/mL;
the volume ratio of the ITS cell culture additive in the culture medium is 1:30-1:300;
the concentration of Y27632 is 3-30 µM);
the concentration of dexamethasone is 0.1-1 µM);
the concentration of Neuregulin-1 is 1-25 ng/mL;
the concentration of insulin is 1-10 µg/mL;
the concentration of the epidermal cell growth factor is 2-18 ng/mL;
the volume ratio of GlutaMAX in the culture medium is 1:30-1:300;
the non-essential amino acids are one or more selected from the group consisting of glycine, alanine, asparagine, aspartic acid, glutamic acid, proline and serine, and the concentration is 50-200 µM.

7. The culture medium of any one of claims 1-5, **characterized in that**, further comprising:
an initial medium selected from the group consisting of DMEM/F12, DMEM, F12 or RPMI-1640; and
one or more antibiotics selected from the group consisting of streptomycin/penicillin, amphotericin B and Primocin.

8. The culture medium of any one of claims 1-5, **characterized in that**, being free of Wnt agonists, R-spondin family proteins, Noggin proteins, BMP inhibitors, or fibroblast growth factor 10.

9. A method for culturing hepatoma organoid, **characterized in that**, comprising the following steps:
(1) isolating samples from solid liver cancer tissue to obtain primary liver cancer cells;
(2) preparing the culture medium for hepatoma organoid according to any one of claims 1-8, and performing organoid culture on the primary liver cancer cells obtained in step (1).

10. A method for evaluating or screening a drug for treating liver cancer, **characterized in that**, comprising the following steps:
(1) culturing hepatoma organoid by using the method for culturing hepatoma organoid according to claim 9;
(2) selecting the drug to be tested and diluting into required concentration gradients;
(3) adding the drug which has been diluted to the organoid obtained in step (1);
(4) detecting the size or the viability of the organoid.
